# EUROPEAN PATENT APPLICATION

(11) **EP 4 640 278 A1**
(43) Date of publication of application: **29.10.2025**
(21) Application number: 24305634.8
(22) Date of filing: 24.04.2024
(51) Int. Cl.: A61P 31/04, A61K 39/00

(54) **FISH VACCINE AND METHOD OF USE**

(71) Applicant: microXpace, 91058 Évry-Courcouronnes Cedex (FR); AquaBIOTEK SPA, 4100000 Chiguayante (CL)
(72) Inventor: OBREGON ALVAREZ, Dasiel, 91058 Évry-Courcouronnes (FR); GALLARDO ESCÁRATE, Cristian, 4100000 Chiguayante (CL); VALENZUELA MUÑOZ, Valentina, 4100000 Chiguayante (CL)
(74) Representative: Flesselles, Bruno F.G.

(57) **Abstract**

The invention pertains to fish vaccines against parasitic copepods, using bacterial products obtained from bacteria of the microbiota of the copepods.

## Description

The invention pertains to the field of veterinary vaccines, notably fish vaccines against parasitic copepods.

Aquaculture is a rapidly growing industry, where one of the primary aims is to maintain the cultured fish's health and welfare. Diseases caused by bacteria, viruses, and parasites can threaten this sector. Traditional vaccines have certain limitations and may not provide broad-spectrum protection. There is a need for innovative vaccines that are safe, effective, and environmentally friendly. The present invention provides a fish vaccine comprising microbiota products derived from the symbiotic (endobiotic (bacteria from the internal organs, tissues, or digestive tracts of copepods) or exobiotic (bacteria that live on the outer surface of the copepod, including their chitinous exoskeletons and appendages)) bacterial community associated with sea lice. The sea lice microbiota products stimulate the fish's immune response, targeting key bacteria in the associated microbiota of sea lice, thus producing dysbiosis in the parasite. The anti-sea lice microbiota vaccine is safe, environmentally friendly, and significantly reduces the parasitic load after 75 days post-immunization. The vaccine can be administrated by injection, orally, or as an in-feed additive.

### BACKGROUND TO THE INVENTION

Salmon aquaculture is a critical economic activity in countries like Norway, Canada, the United Kingdom, and Chile. However, this industry faces various challenges, including sea lice infestations, which have become a significant concern due to their impact on fish health and aquaculture sustainability. Sea lice infestations, primarily from the *Caligidae* family, cause skin damage to salmon, leading to chronic stress and reduced growth rates. This problem exacerbates production costs, with delousing treatments adding approximately USD 1.4 per kilogram of salmon produced. However, the industry's reliance on chemical treatments' reduced efficacy underscores the pressing need for novel and effective management strategies.

This disease is caused by the sea lice species belonging to the *Caligidae* family, and the most prevalent species in salmon farming are *Caligus rogercresseyi* and *Lepeophtheirus salmonis.* The life cycle of *C. rogercresseyi* includes eight different developmental stages involving three free-living larval stages followed by five parasitic stages. During the parasitic stages, the sea lice feed on the mucus and skin of its hosts, producing skin damage that can lead to chronic stress, reducing feed conversion rates and growth in farmed salmons. Heavy lice infections also lead to erosion of the epidermis with exposure of the dermis and, in severe cases, skeletal muscle. Physiological consequences of infections, including stress, changes in blood glucose or electrolytes, reduced hematocrits and reduced swimming performance can also be observed, such effects being notably influenced by the number of copepods and their stage of development. The parasitic stages are associated with complex biological and molecular responses triggered by the host and parasite during their interaction.

Recent genomic studies have revealed a diverse microbial community associated with sea lice species, both externally and internally. For example, the microbiota of *C. rogercresseyi,* collected from various populations, has been extensively characterized (Gonçalves et al, Sci Rep. 2020; 10: 2895). This analysis identified several taxa, including members of the genera *Pseudoalteromonas* and *Dokdonia* (Gonçalves et al, *op.cit.*)*.*

The genome of *Dokdonia sp.,* one of the most represented in the sea lice microbiome, encodes genes crucial for the biosynthesis and degradation of essential amino acids such as valine, leucine, and isoleucine.

The limitations of current chemical-based methods drive the quest for innovative sea lice management strategies. Adjusting factors like water temperature, salinity, and habitat structure in aquaculture settings could potentially disrupt sea lice breeding and survival rates, providing a more natural control method. However, these methods may need more efficient to be industrially viable in aquaculture.

The invention shows that targeting and disrupting the relationship between sea lice and their microbiota impairs their ability to infect and thrive on salmon hosts. Using a vaccine aiming at producing a fish immune response against bacteria in the microbiota of sea lice makes it possible to reduce infestations. It is hypothesized that such observation is due to interference with critical metabolic processes of the lice, which makes them unable to grow properly on the fish.

Hence, the parasite induces an immune response in the host that, once infected by the parasite, disturbs key microbial species within the sea lice microbiota, promotes a hostile environment for the lice, and reduces their capacity for reproduction and survival.

This strategy promises a targeted approach to controlling sea lice populations and aligns with sustainable aquaculture practices by reducing reliance on chemical treatments.

The invention thus relates to a fish vaccine against a parasitic copepod, comprising bacterial products isolated from bacteria of the microbiota of the parasitic copepod.

The fish vaccine shall be presented in a veterinary-acceptable formulation (containing excipients acceptable and usable for veterinary use) and may also contain adjuvants usable for veterinary use. Several adjuvants are already used in aquaculture vaccines, such as mineral oil emulsions or oil-in-water emulsions (notably based on saponins), aluminum hydroxide, mineral salts (such as aluminum phosphate and calcium phosphate), bacterial cell wall components (e.g., lipopolysaccharides), and polymeric adjuvants (such as chitosan and poly(lactic-co-glycolic acid) (PLGA)). Excipients that can be used include buffers (for maintaining vaccine pH within a suitable range), stabilizers (such as sugars (e.g., sucrose, mannitol) and proteins (e.g., gelatin)), surfactants (such as polysorbate 80 and Tween^{®} 80, to improve wetting, dispersibility, and solubility of the antigen or stabilize emulsions and suspensions), preservatives (to prevent microbial growth and contamination during storage and use; one can cite thimerosal, benzyl alcohol or formaldehyde), diluents (such as saline solutions or other buffering agents), antioxidants (which can prevent oxidation; one can cite ascorbic acid (vitamin C) and alpha-tocopherol (vitamin E)), or various carriers for delivering the vaccine formulation (such as liposomes, nanoparticles, or other particulate carriers).

The bacterial products used in the vaccine against the copepod may be selected from attenuated bacteria, inactivated bacteria, or bacterial extracts. The bacterial extracts are derived from bacterial cells or culture supernatants. They contain a mixture of various bacterial components such as bacterial membrane components, proteins, polysaccharides, nucleic acids, lipids, and metabolites (notably immunogenic metabolites). As bacterial extracts, one can specifically mention bacterial lysates (obtained by lysing bacterial cells and which contain a mixture of intracellular components such as proteins, DNA, RNA, and metabolites), bacterial polysaccharides (complex carbohydrates present in the cell wall or the capsule of bacteria), bacterial cell wall components (such as peptidoglycan, lipopolysaccharide (LPS), and lipoteichoic acid), bacterial metabolites (which can have immunogenic or immunomodulatory effects; one can cite lipids and short-chain fatty acids).

The products are obtained from bacteria isolated from the microbiota of the parasitic copepod. In particular, the sea lice bacteria can be isolated from adult parasites infecting fish. As disclosed below, the bacteria can then lysed or inactivated to obtain the products, that are used and formulated into the vaccine.

It is preferred when the bacteria from which the products are isolated present a symbiotic effect with the copepod, in particular a mutualistic relationship, where they aid in the digestion of food, provide essential elements (such as nutrients or essential amino acids), or protect the copepod for harmful organisms, and hence provide benefit to the copepod.

These products may include bacterial cell components, metabolites, and secreted proteins. They can be obtained after the bacteria are cultured under conditions that induce the production of immunostimulatory compounds.

In one embodiment, the bacteria are endobiotic bacteria. Endobiotic bacteria may produce enzymes lacking from the copepod (this can be determined by comparing the genomes of the bacteria and copepod; Tang. Aquat. Microb. Ecol. 2005, 38, 31-40), be able to digest complex organic matter, such as phytoplankton or detritus, and to break down these substances into more easily digestible forms for the copepod or synthesize vitamins and cofactors (Gorokhova et al, Front. Microbiol. 2021, 11, 589816).

In another embodiment, the bacteria are exobiotic bacteria. Exobiotic bacteria can deter harmful pathogens or assist in the copepod's defences. In addition, some exobiotic bacteria can produce antimicrobial compounds that help protect copepods from harmful pathogens, parasites, or fouling organisms, for example, by inhibiting the attachment and growth of these organisms.

In some embodiments, endobiotic and exobiotic bacteria are used as a source of products usable in the vaccine.

Among bacteria that are in symbiosis with copepods, one can cite the *Vibrio spp.,* which produces essential amino acids such as lysine and methionine and can also synthesize and provide B vitamins. One can also cite bacteria of the *Pseudoalteromonas spp.,* which produce various amino acids, including glycine, serine, and proline, and can assist copepods in digesting organic matter.

One can also cite bacteria of the genus *Dokdonia,* notably *Dokdonia donghaensis.* Dokdonia bacteria are Gram-negative bacteria and play roles in organic matter degradation and nutrient cycling. They are heterotrophic and hence have the ability to degrade complex organic matter, including polysaccharides and proteins, to obtain their required nutrients. In particular, when present in the microbiota of copepods, they can degrade organic components from the ingested food of the copepods and produce simpler components that can, in turn, be used in their metabolism. Such bacteria thus have a mutualistic relationship with the copepod. Dokdonia bacteria include *D*. *diaphoros, D. donghaensis, D. eikasta, D. genika, D. pacifica, D. flava, D. lutea, D. sinensis, D. aurantiaca* and *D. ponticola.*

Other bacteria from the Flavobacteriaceae family are *Cellulophaga* bacteria, which are Gram-negative bacteria that can degrade cellulose into metabolizable compounds. One can cite *Cellulophaga lytica, C*. *geojensis, C*. *algicola, C*. *baltica* and *C*. *fucicola.*

It has also been shown that the genome of bacteria in the microbiota of copepods, notably of Dokdonia and Cellulophaga bacteria, comprise genes coding for the biosynthesis and degradation of essential amino acids such as valine, leucine, and isoleucine. These bacteria are thus of particular interest to be used in the vaccine, as an immune response against such can disrupt metabolism in the copepod.

The genome sequencing of Dockdonia and Cellulophaga bacteria showed genome sizes of 6,415,100 base pairs (bp) and 3,523,379 bp, respectively. Genome annotation revealed 6,512 and 4,737 coding-proteins, and 7 and 6 rRNAs genes, respectively. Concerning the non-coding RNAs, such as tRNAs, the genomes of these bacteria showed 65 and 53 predicted products, respectively.

Identification and characterization of bacteria from the microbiota can be performed according to any suitable method, including culture of bacteria on a solid medium and verification of the nature of the bacteria by sequencing or amplifying 16S rRNA.

In one embodiment, the vaccine comprises products from Dokdonia bacteria, notably from *D*. *donghaensis* bacteria.

In one embodiment, the vaccine comprises products from Cellulophaga bacteria, notably *C*. *baltica, C. geojensis,* and *C. algicola* bacteria, or a mixture of two or three.

In one embodiment, the vaccine comprises products from Aeromonas bacteria, particularly Aeromonas salmonicida. Such bacteria are associated with Lepeophtheirus salmonis (Feng et al., 2013). A vaccine using products from these bacteria may thus be useful for protecting salmon against seal lice and Aeromonas-induced furunculosis.

In one embodiment, the vaccine comprises a mixture of products from Dokdonia bacteria, notably *D*. *donghaensis* bacteria, and of products from Cellulophaga bacteria, notably *C*. *baltica, C*. *geojensis,* and *C*. *algicola* bacteria, or a mixture of two or three of them.

In one embodiment, the vaccine comprises inactivated Dokdonia and/or Cellulophaga (baltica, geojensis, algicola) bacteria (bacteria that cannot divide and grow). The bacteria may be inactivated by heating, irradiation (UV irradiation, X-ray irradiation, gamma or beta-ray irradiation), chemical agents, sonication, highpressure processing, or any other method known in the art. Heating and/or use of chemical agents are particularly appropriate.

In one embodiment, the vaccine comprises cell lysates obtained by cell lysis (notably through mechanical disruption, chemical lysis (using detergents such as Triton X-100 or Tween or chaotropic agents such as urea or guanidine hydrochloride), or enzymatic lysis. As bacterial cell lysate, one can use the whole cell lysate (including the cell debris membranes and insoluble components), a clarified cell lysate (where the cell debris membranes and insoluble components have been removed, for example, by filtration or centrifugation), or the cell debris membranes and insoluble components obtained after lysis.

The vaccines herein disclosed can be used to protect a fish against any copepod, that presents, in its microbiota, the bacteria from which the bacterial product is isolated. Study of the microbiome of copepods to isolate useful bacteria is within the skill of one of skill in the art. Feng et al (Water. 2023; 15(24):4203) reviews studies regarding the microbiome associated with marine copepods, focusing on the diversity of bacteria and fungi and the documents and the specific teachings cited therein are incorporated by reference, notably the teachings confirming that symbiotic *"Bacteria can produce enzymes that copepods themselves may lack, allowing them to access and utilize specific nutrients that would otherwise be inaccessible or difficult to obtain* [and] *can provide other benefits to copepods, such* as *the synthesis of essential vitamins and cofactors that may be lacking in their diet",* and the table describing bacteria that are symbiotic to copepods. Skovgaard et al (PLoS One. 2015; 10(7): e0132516) describe the characterization of microbiome of copepods, using 16S rRNA analysis. Gerdts et al (Helgol Mar Res 67, 757-773 (2013)) describe analysis and comparison of the microbiome of various copepods using 16S rRNA sequencing.

In a preferred embodiment, the copepod is a sea louse (in particular from the *Lepeophtheirus* or *Caligidae* families). In one embodiment, the copepod is *Caligus rogercresseyi.* In another embodiment, the copepod is *Lepeophtheirus salmonis.* In one embodiment, the vaccine is able to protect the fish against both *Caligus rogercresseyi* and *Lepeophtheirus salmonis,* when bacterial products from the microbiome of both these organisms are used in the vaccine.

In a preferred embodiment, the fish is a salmon. The vaccine is particularly interesting in aquaculture farms growing salmon of the *Salmo* or *Oncorhynchus* genera. One can cite *Salmo salar* (Atlantic salmon, grown in Northern Europa, such as in Norway, Ireland, Scotland, Feroe Islands), or Pacific salmon (*Oncorhynchus tshawytscha, O. keta, O. kisutch, O. masou, O. gorbuscha* or *O. nerka*)*.*

In one embodiment, the vaccine is formulated for oral administration. The bacterial products can be mixed with the animal's food, whether in crude form or encapsulated in various vehicles. Techniques include using adhesives like edible oil or gelatin, spraying the formulated food with a liquid vaccine suspension, and co-processing the antigen with aquafeed during manufacturing. One can also cite encapsulation techniques for better protection and immune response, using alginate, chitosan, or poly D, L-lactic-co-glycolic acid (PLGA) encapsulation. It is worth noting that Mutoloki et al (Front Immunol. 2015; 6: 519) review methods for formulating oral vaccines for fish, as does Embregts et al (Developmental and Comparative Immunology 64 (2016) 118e137). Currently, available oral vaccines include live attenuated vaccines (against bacterial kidney disease (BKD), *Flavobacterium columnare,* viral haemorrhagic septicaemia virus (VHSV) vaccine, a viral vaccine against Koi herpesvirus (KHV) for carp and attenuated *V. anguillarum* vaccine in rainbow trout), inactivated vaccines against *V. anguillarum, V. ordalli*, *Y. ruckeri, V. salmonicida,* and *A. salmonicida.* One of skill in the art may thus formulate a vaccine for oral administration using the bacterial products of the microbiome-isolated bacteria.

In one embodiment, the vaccine is formulated for immersion administration. This method is based on the fact that skin epithelium and gills present mechanisms that can protect fish so that mucosal surfaces can recognize pathogens with whom they have been in contact. This method consists of immersing fish in water containing the (diluted) vaccine so that the skin and gills may adsorb the antigens in the suspension. In the dipping method (dip vaccination), fish are immersed for a very short duration, usually 30 seconds, in a highly concentrated vaccine solution, usually 1 part vaccine product to 9 parts water. In bath vaccination, fish are exposed to a more diluted vaccine solution for a more extended period, usually one to several hours. In large fish farms, dip vaccination may be preferred.

In one embodiment, the vaccine is formulated for administration by injection. This method may not be favored, as it is burdensome (notably for large farms) and may induce stress from the handling and injection, which may cause fish mortality. It may thus be advised to use light anesthesia of fish. Injection may be performed intramuscularly or intraperitoneally. However, compared to immersion and oral administration, this makes it possible to finely control the amount of vaccine administered to the fish.

It is preferred that the vaccine is administered to fish between 80 and 120 grams of total weight.

In some embodiments, a single administration is performed, preferably on fish during the freshwater stage denominated "parr" (10 and 25cm in body length).

In other embodiments, multiple administrations (a first shot + booster(s)) are performed. Preferably, the first shot is performed on the parr stage, and boosters (generally one booster) are administered 6 months later by oral or intraperitoneal injection.

The amount of bacterial product can be determined based on the amount of bacteria before preparing the products (before inactivation, lysis...). Determining optimal doses are within the skills of one of skill in the art. As an illustration, it is preferred to administer an amount of bacterial product in the vaccine obtained from Dokdonia and Cellulophaga bacteria in doses prepared from between 1×10⁵ and 1×10⁷ (preferably around 1×10⁶) and between 1×10⁹ and 1×10¹¹ (generally about 1×10¹⁰) cells in 100 µL of formulation for inactivated and live vaccines, respectively.

The invention also relates to a method for producing a fish vaccine against a parasitic copepod, comprising mixing bacterial products isolated from the bacteria of the parasitic copepod's microbiota (microbiome) with an excipient acceptable for veterinary use (as described above) to obtain a formulation that can be administered to fish.

The invention also relates to a method for protecting a fish (or a population of fish) against a parasitic copepod, comprising administering the fish vaccine as herein disclosed. The vaccine may be used for therapeutic effect (administered in case of infection of fish by the copepod, notably in case of salmon infection by sea lice), or for a prophylactic effect (avoiding infection of fish by copepods). It is to be noted that the effect may be partial. The prophylactic effect may not fully prevent infestation but may reduce the effect of such (for instance, fewer copepod per fish, fewer symptoms for the fish, as compared to the expected effects, should the fish not have been vaccinated). The therapeutic effect may not lead to all copepods unhooking from the fish, but to some of them, or to a reduction in the copepod infection intensity.

In view of the composition of the vaccine, a bacterial product from bacteria of the microbiota (microbiome) of a parasitic copepod can be used to protect (therapeutically or prophylactically) a fish against infection by the parasitic copepod, and a method thereof can be implemented.

The vaccines use bacterial products from bacteria that are symbiotic to the copepods. Such bacteria, usable in a fish vaccine herein disclosed, can be identified by a method comprising
a) Isolating bacteria from the microbiota of the parasitic copepod
b) Sequencing bacterial genome of the bacteria isolated in a)
c) Identifying genes from the bacterial genome that complement missing or deficient pathways in the genome of the parasitic copepod.

The bacteria can be isolated by crushing the copepod in a liquid (such as saline), centrifuging the solution, and spraying the supernatant on a nutritive solid medium.

The morphology of colonies can then be studied, and the genome of the colonies can be obtained by sequencing. High-throughput-sequencing can be used, and bioinformatics analysis makes it possible to reconstitute the bacteria's genome and identify genes that are important for synthesizing metabolites that are not synthesized from genes of the copepod's genome. Thus, it can be assumed that these genes would complement missing or deficient pathways of the genome of the parasitic copepod and that the bacteria bearing these genes are good candidates for the vaccine. The sequence of the 16S rDNA (or 16S rRNA) bacteria can be identified and later used to verify the presence of the target bacteria in further samples.

In summary, the anti-microbiota vaccine formulation provides prevention and/or treatment of parasitic copepod infections in fish, notably in salmonids infected by sea lice species. The vaccine uses bacterial products from the microbiota of sea lice. It is believed to work by redirecting the host fish's immune response towards targeting the parasite microbiota, which in turn causes dysbiosis in the symbiotic bacterial community associated with parasitic copepods. This ultimately leads to a high mortality rate of lice infecting the immunized hosts.

Fish are preferably salmonids, such as *Salmo salar, Oncorhynchus mykiss, Oncorhynchus kisutch, Salmo trutta,* or *O. tshawytscha;* and the copepods are preferably *Caligus rogercresseyi, Caligus elongatus* or *Lepeophtheirus salmonis.* As shown in the examples, the immune response of vaccinated fish to sea lice infection shows up-regulation of genes related to Th2 response. The microbial diversity and abundance in sea lice exposed to immunized fish reveal significantly low values compared with the bacterial communities collected from unvaccinated fish. The anti-sea lice microbiota vaccine is safe and environmentally friendly and reduces the parasitic load by 80% after 90 days post-immunization. The vaccine can be administrated by injection, orally, or as an in-feed additive.

### FIGURES

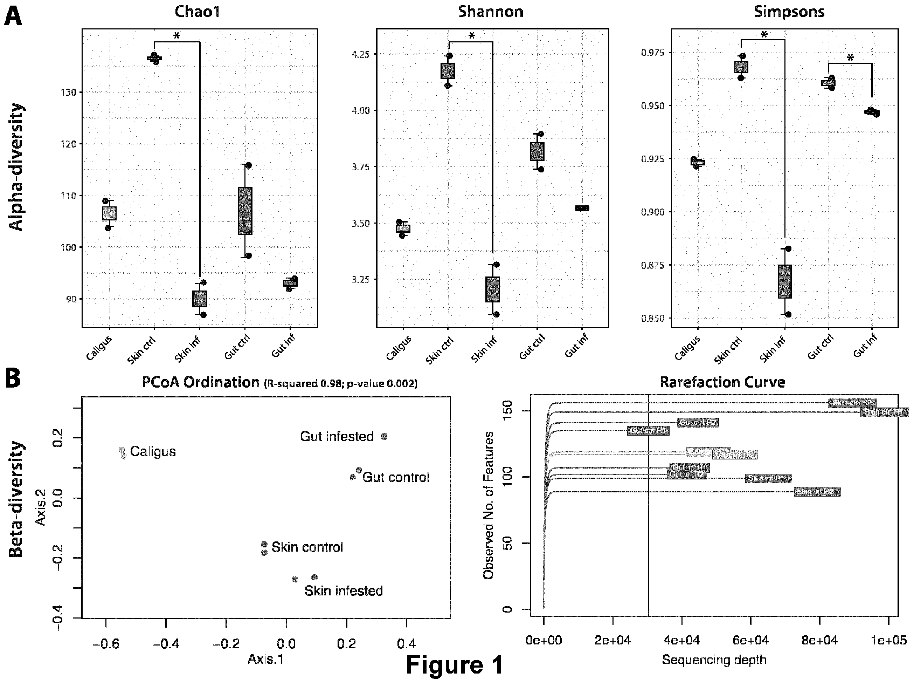

Figure 1: Figure 1 shows the microbial community diversity in the skin, gut, and microbial community diversity in the sea louse C. rogercresseyi of infected and uninfected Atlantic salmons. A) The alpha-diversity index includes Chao1, Shannon, and Simpsons. * Denotes statistically significant differences (p-value < 0.05). B) Beta diversity, including Principal Coordinates Analysis (PCoA) and Rarefaction curve for all sequenced samples.

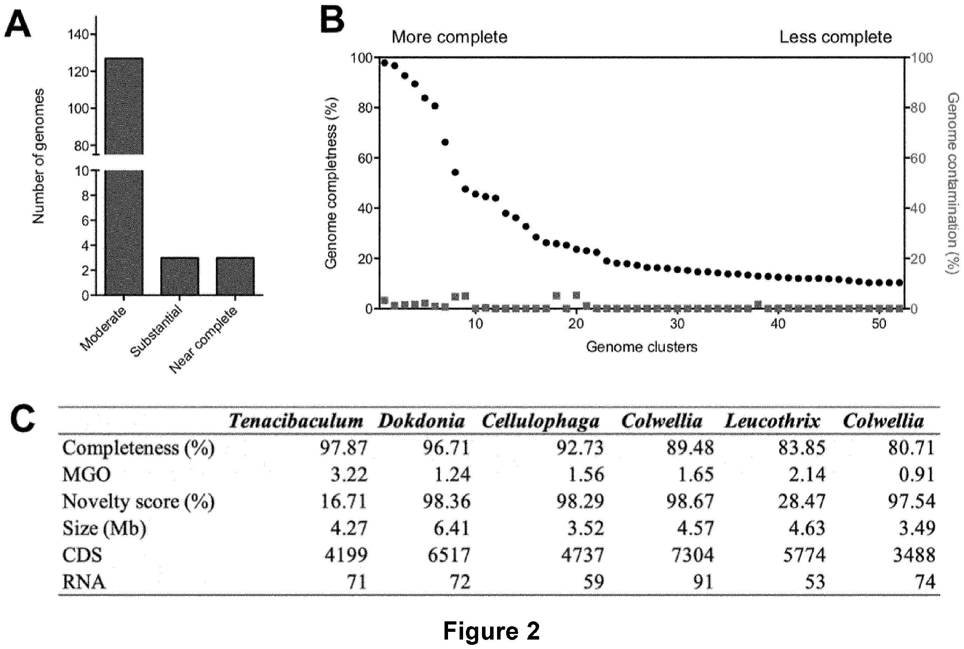

Figure 2: Figure 2 summarizes the genomic reconstruction of bacterial clusters associated with the sea louse *Caligus rogercresseyi.* A) Genome cluster classification according to their completeness in near complete (>95%), substantial (>80%), and moderate (<80%). B) Genome completeness and contamination among the 50 most represented bacterial clusters. C) Main genomic features of the top 5 represented genome clusters in sea lice microbiota.

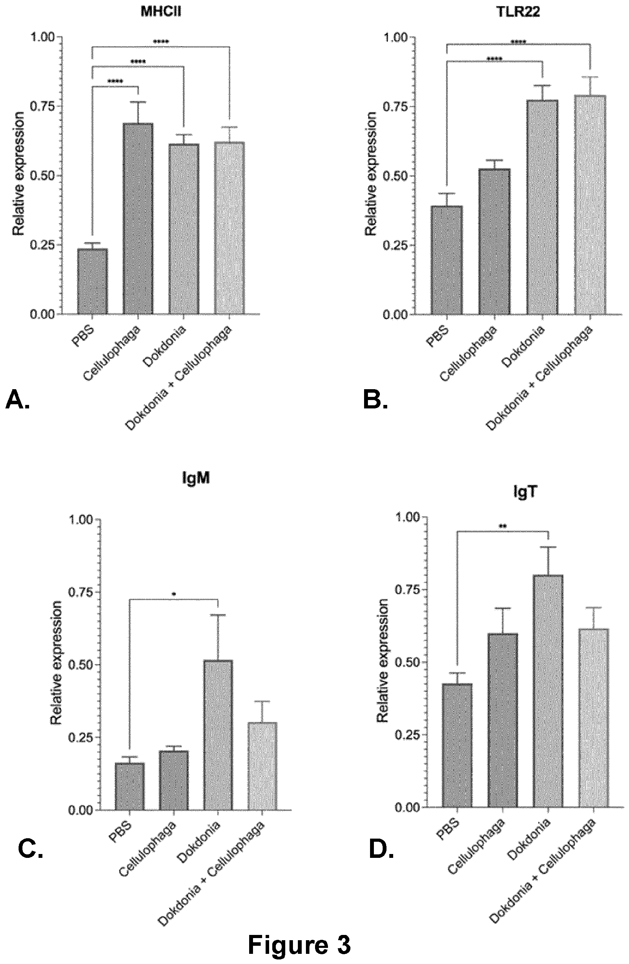

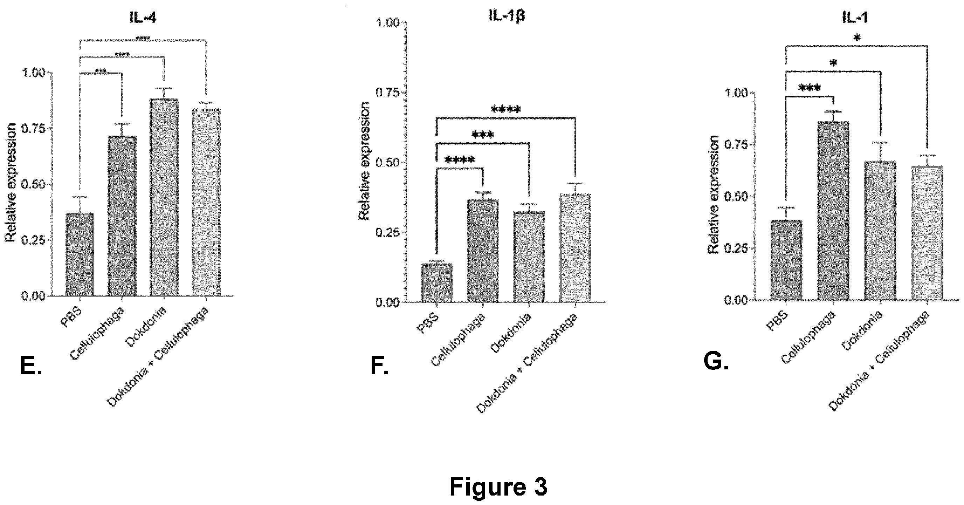

Figure 3: Figure 3 shows in graphs the Atlantic salmon immune response induced by the anti-microbiota vaccines. Relative expression was estimated using the β-tubulin gene as housekeeping. The specific transcription expression levels were measured by RT-qPCR. A. MHC II molecules; B. TLR22; C. IgM; D. IgT; E: IL-4; F: IL-1β; G: IL-1.

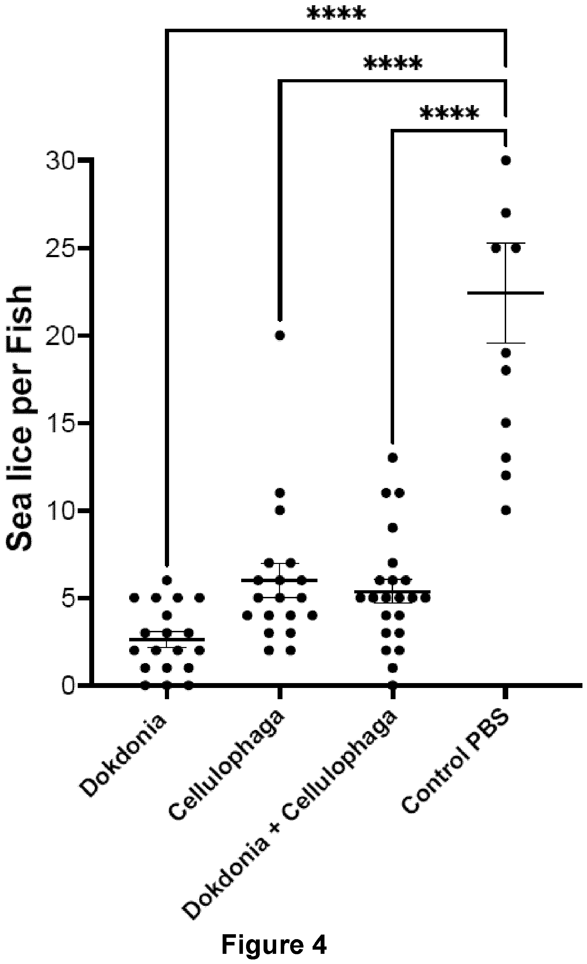

Figure 4: Figure 4 shows in a graph the efficacy of the anti-microbiota vaccines as the number of sea lice per fish. The anti-microbiota vaccines were formulated as inactivated (killed) bacterial (bacterins) compositions for Dokdonia, Cellulophaga, and a mix. The parasitic burden was evaluated after two successive infections at 72 days post-infection, equivalent to 1600 accumulated thermal units (ATU). The control fish group was injected with PBS. Significant differences (*) were estimated for a p-value <0.0001.

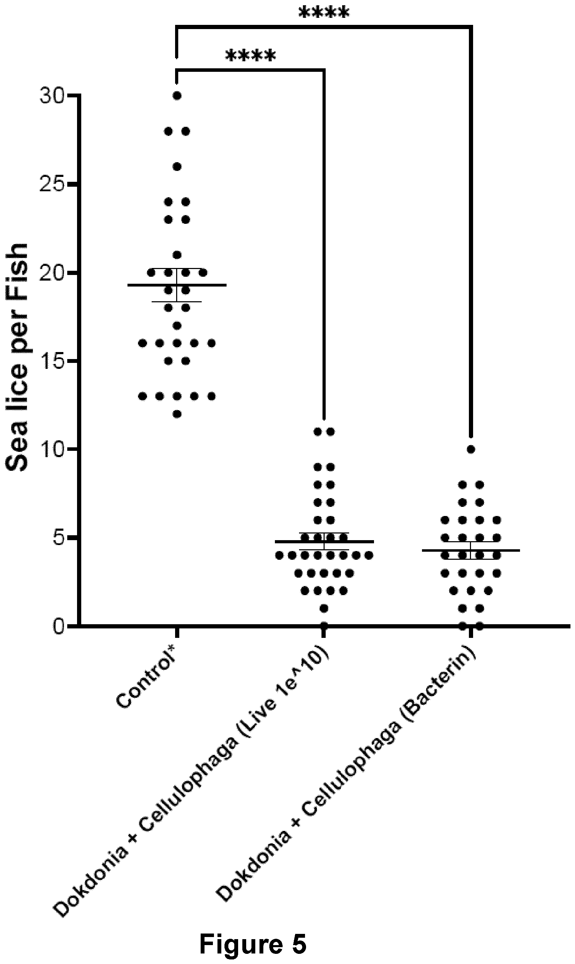

Figure 5: Figure 5 shows in a graph the efficacy of the anti-microbiota vaccines as the number of sea lice per fish. The anti-microbiota vaccines were formulated as bacterins or inactivated mix of Dokdonia and Cellulophaga. A live vaccine was also formulated with the same bacterial composition in a concentration of 10¹⁰ cells. The parasitic burden was evaluated after two successive infections at 50 days post-infection. The control fish group was injected with PBS. Significant differences (*) were estimated for a p-value <0.0001.

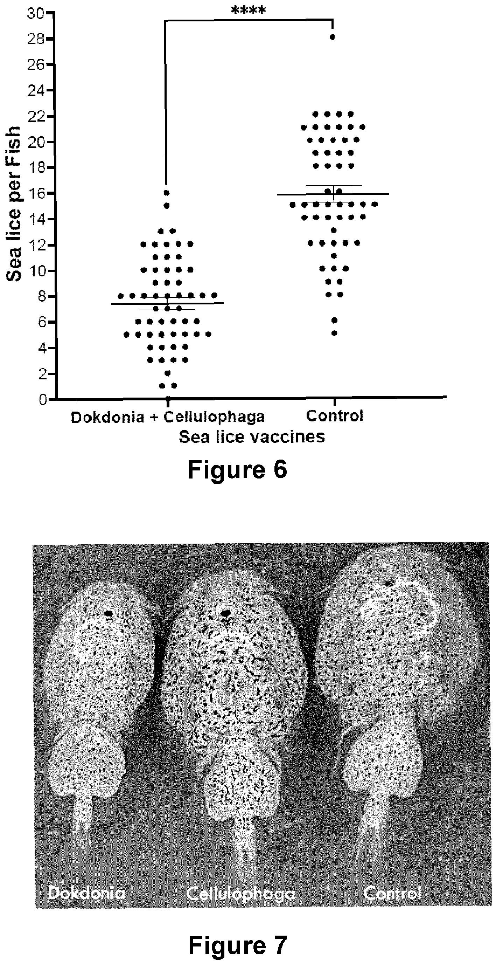

Figure 6: Figure 6 shows in a graph the efficacy of the anti-microbiota vaccine administrated by oral delivery. The efficacy was evaluated by the number of sea lice per fish. The anti-microbiota vaccine was formulated as a combination of Dokdonia and Cellulophaga live bacteria, using a final concentration of 10¹⁰ cells per gram of commercial fish feed as an in-feed additive. The parasitic burden was evaluated after 25 days post-infection. A commercial formulation was used as control fish group (without in-feed additives or probiotics). Significant differences (*) were estimated for a p-value <0.0001.
Figure 7: Figure 7 shows a photograph of three adult females collected from Atlantic salmon immunized with Dokdonia, Cellulophaga, and control vaccines. Differences in pigmentation of the abdominal segment and body size were observed among lice collected from immunized fish.

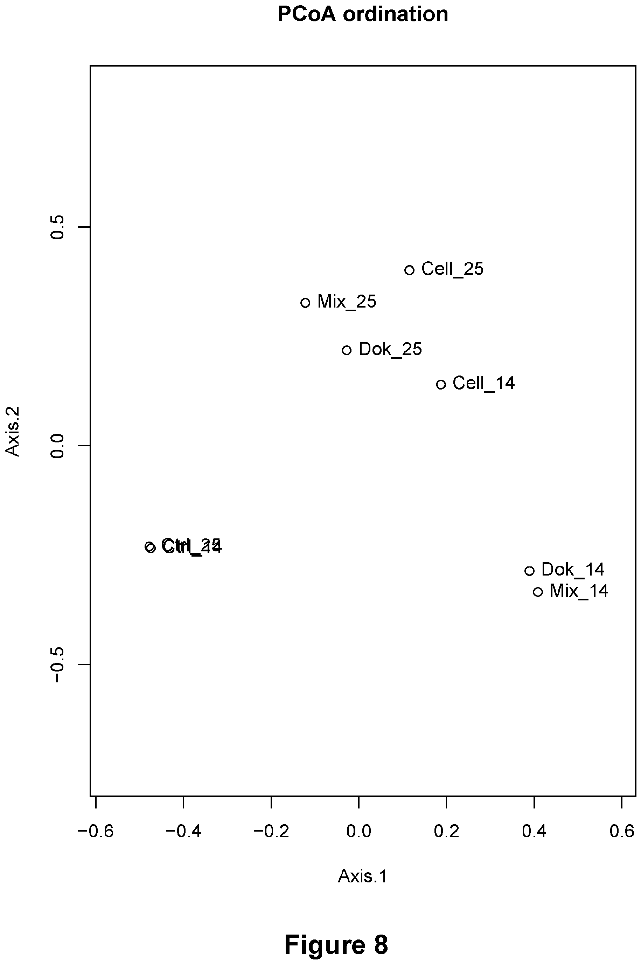

Figure 8: Figure 8 shows the Principal Coordinates Analysis (PCoA) for the microbial community diversity in *C. rogercresseyi* collected from immunized fish with anti-microbiota vaccines. The formulations were prepared with either inactivated Dockdonia, Cellulophaga, or a mix of these bacteria. The microbial community evaluation was performed at 14 and 25 days post-infection. The PcoA shows significant dysbiosis of lice microbiota when the parasites are exposed to fish immunized with antimicrobiota vaccines. The evaluation of the bacterial community revealed that at 14 dpi, lice collected from immunized fish with Dokdonia and Dokdonia + Cellulophaga (mix) display similar patterns not associated with the control group. The bacterial diversity at 25 dpi showed a significant cluster composed by Cellulophaga, Dokdonia and Dokdonia + Cellulophaga (mix).

### EXAMPLES

### Example 1. Sea lice DNA isolation

Sea lice and salmon DNA as isolated using the Qiagen DNA purification kit (QIAGEN, Germantown, MD, USA) according to manufacturer's instructions. DNA quality and integrity was assessed through a 1% agarose gel and NanoDrop 1000 Spectrophotometer. Samples with no smear and absorbance ratios above 1.8 were used for further processing.

From isolated DNA, nanopore sequencing libraries were prepared to amplify the full 16S rRNA gene as known in the art. Briefly, DNA isolated from all the experimental groups were used for PCR using the universal specific primers for the amplification of the full-length 16S rRNA gene 27F 5'-AGAGTTTGATCCTGGCTCAG-3' (SEQ ID NO: 1) and 1492R 5'-GGTTACCTTGTTACGACTT-3' (SEQ ID NO: 2), with Taq DNA polymerase LongAmp (NewEngland Biolabs, USA) using the standard PCR program. PCR products can be then quantified by Qubit 4 (Thermo Scientific, USA) and used for nanopore library preparation using the 16S Barcoding Kit (SQK-RAB204, Oxford Nanopore Technologies) according to manufactures instructions. Different barcodes were used for each sample and each sample will be sequenced in triplicate in a Nanopore MinION platform (Oxford Nanopore Technologies, UK) using the MK1 Spot-ON FLO-MIN107-R9 flowcell. As an internal normalizer, a library prepared from DNA isolated from a mock community (ZymoBiomics Microbial Community Standard) was also included.

After basecalling and quality filter, the reads were demultiplexed and trimmed using the Fastq 16S (Oxford Nanopore Technologies, UK) pipeline with default parameters. Filtered reads will be initially annotated through Blastn against the NCBI RefSeq for 16S ribosomal RNA database using the EPI2ME software (Oxford Nanopore Technologies).

For the assignment of each bacterial species, the 16S rRNA gene reads longer than 1,400 nt and with an accuracy above 85 % were considered for further analysis. The frequency of each sample was calculated in R statistical software [105] and ordered as an Operational Taxonomic Unit (OTU) table. Simpson's and Shannon index will be used to estimate alpha diversity, while Pielou's index was used to estimate evenness. All indexes will be calculated using R's "Vegan" package. Moreover, proximity ligation libraries were used to reconstruct the bacterial genomes in the microbiota. Shotgun metagenomic libraries were prepared using the TruSeq DNA PCR-Free Library Prep kit (Illumina, San Diego, CA, USA), while Hi-C sequencing libraries were prepared using the Phase Genomics' Animal Hi-C kit (Phase Genomics, Seattle, WA, USA) following the manufacturer's instructions. Both libraries were sequenced using HiSeq 4000 (Illumina, San Diego, CA, USA). Sequence reads were mapped against the last version of the sea lice genome or the Atlantic salmon genome using the BWA-MEM alignment tool (Li et al, 2013, doi: 10.48550/arXiv.1303.3997). Unmapped reads were de novo assembled using Megahit (Li et al, Bioinformatics, 31 (10), 2015, 1674-1676) and assessed through MetaQuast (Mikheenko et al, Bioinformatics. 2016 Apr 1;32(7):1088-90). The Hi-C reads were mapped against the *de novo* assembly through the Burrows-Wheeler alignment tool BWA-MEM. The deconvolution of contigs was performed. Contigs were grouped into genome clusters considering the Hi-C contact data using a proprietary Markov Chain Monte Carlo algorithm. The quality control of the bacterial genome cluster were assessed using CheckM (Parks et al, Genome Res. 2015 Jul;25(7):1043-55). Thus, genome clusters with marker gene overrepresentation (MGO) over five were discarded from subsequent analysis. Genome annotation was performed using the Rapid Annotations using Subsystems Technology (RAST) servers.

### Example 2. Site selection and sea lice collection

Parasites were collected from commercial salmon farms located in Los Lagos, Aysén and Magallanes regions in Chile during winter and summer. Samplings were conducted under the standardized protocols.

Briefly, Atlantic salmons (*Salmo salar*) were confined in sea cages using secondary nets, being collected with a soft net and sedated in a reservoir previously loaded with Benzocaine (20%) in seawater (15ml per 100L seawater).

After this, sea lice were collected with plastic sterilized forceps. *C. rogercresseyi* destinated to DNA isolation are going to be transferred to vials with molecular grade ethanol. Vials were stored at -20°C and transported in cooled boxes by overnight express service to the laboratory where they were stored at -80°C until processing.

The identification of keystone taxa was performed and based on co-occurrence microbial networks. For this, the microbial profiling data was generated for *C. rogercresseyi,* and the co-occurrence networks was constructed for each dataset based on taxonomic profiles. The SparCC method implemented in the R environment was used to calculate the correlations matrix. The topological parameter of the networks (i.e., number of nodes and edges, weighted degree, diameter of the network, modularity, and clustering coefficient) were calculated in each data set. Furthermore, centrality metrics (i.e., betweenness centrality, harmonic centrality, and closeness centrality) was estimated for measure the keystoneness of each node, and selected eigenvector centrality for the analysis. Calculations and network visualizations was done with the software Gephi.

### Example 3. Anti-microbiota vaccine formulation

The results obtained from examples 1 and 2 were used to identify the keystone bacterial taxa.

These bacterial candidates were used for the formulation for the anti-microbiota vaccine. For bacterial isolation, lice were washed with PBS and mechanically crushed, the supernatant was diluted and transferred to a culture media depending on the nutritional requirements of the isolated bacteria.

After incubation, the liquid media was seeded on plates to identify discrete colonies that was isolated and used for DNA isolation. Finally, universal 16S primers were used to amplify this gene through PCR and the products were sequenced by sanger sequencing.

The obtained sequences were annotated against NCBI 16S data base to confirm the bacteria specie.

Inactivated vaccines were prepared from Cellulophaga, Dokdonia, and a mix of them.

Briefly, the bacteria inactivation was carried out using separated suspensions of 1 × 10⁹ cells of each bacteria in marine broth (76448 Marine Broth 2216, Sigma-Aldrich, USA), and incubated at 100°C for 30 min. Then, 1% formalin v/v was added and incubated at 4°C for 24 hours. Subsequently, the suspensions were centrifuged at 10,000g for 10 minutes at 4°C, and two washing steps were carried out with 1X PBS. The bacteria pellets were resuspended in 1X PBS, with a final concentration of 1×10⁶ cells for each species. Next, 2V of Montanide ISA 761 VG (Seppic, Paris, France) were adjuvanted to both suspensions. Additionally, a mix of bacteria, Cellulophaga and Dokdonia, were formulated in proportions of 50% of each bacterium at the same final concentration. In addition, a control vaccine was formulated with PBS and adjuvant.

Vaccines were formulated in 100 µL per dose in a ratio of 30% inactivated bacteria to 70% adjuvant (Montanide). Atlantic salmon (n=20) were IP injected with 100 µL of the vaccine for each vaccine. After 400 ATUs, skin and head kidney samples were collected to analyze the molecular responses of Atlantic salmon to each vaccine prototype. The infection outcome was measured (% Adults), and lice samples were collected to analyze changes in the parasite's microbiota.

In other experiments, the vaccine was administered to fish as an in-feed additive at a dose of 10¹⁰ cells per gram of commercial fish feed.

This example shows that the vaccine can be administered via injection, or oral delivery. The optimal dosage and administration frequency can be adjusted to the fish species and the prevailing environmental conditions.

**Storage and Handling:** The vaccine was easy to produce and can be stored at room temperature for at least two months, even at least three months, and at least six months.

**Safety and Efficacy Trials:** The vaccine provides broad-spectrum protection against various sea lice species, and is also expected to provide side protection effects in co-infection with pathogenic bacteria. The vaccine was shown to be safe, with no observed adverse effects on fish health.

## Claims

1. A fish vaccine against a parasitic copepod, comprising a bacterial product isolated from bacteria of the microbiota of the parasitic copepod.

2. The fish vaccine of claim 1, wherein the product isolated from bacteria of the microbiota of the parasitic copepod comprises attenuated bacteria, inactivated bacteria, or bacterial extracts.

3. The fish vaccine of claim 1 or 2, wherein the bacterial product is inactivated bacteria.

4. The fish vaccine of any one of claims 1 to 3, wherein the bacteria present a symbiotic effect with the copepod.

5. The fish vaccine of any one of claims 1 to 4, wherein the bacteria produce enzymes lacking from the copepod or can digest complex organic matter and to break down these substances into more easily digestible forms for the copepod or synthesize vitamins and cofactors.

6. The fish vaccine of claim 1 or 5, wherein the copepod is a sea louse, in particular from the *Lepeophtheirus* or *Caligidae* species, in particular *Caligus rogercresseyi* and *Lepeophtheirus salmonis.*

7. The fish vaccine of any one of claims 1 to 6, wherein the fish is a salmonid.

8. The fish vaccine of any one of claims 1 to 7, wherein the bacteria comprise *Dokdonia* or *Cellulophaga* bacteria.

9. The fish vaccine of any one of claims 1 to 8, which comprises inactivated *Dokdonia* bacteria, inactivated *Cellulophaga* bacteria or a mix of *Dokdonia* bacteria and inactivated *Cellulophaga* bacteria.

10. The fish vaccine of any one of claims 1 to 9, which is formulated for administration by injection, immersion or oral administration.

11. A method for producing a fish vaccine against a parasitic copepod according to any one of claims 1 to 10, comprising mixing bacterial products isolated from the bacteria of the microbiota of the parasitic copepod with an excipient to obtain a formulation that can be administered to fish.

12. A method for identifying bacteria from the microbiota of a parasitic copepod, usable in a fish vaccine according to any one of claims 1 to 10, comprising
a) Isolating bacteria from the microbiota of the parasitic copepod
b) Sequencing bacterial genome of the bacteria isolated in a)
c) Identifying genes from the bacterial genome that complement missing or deficient pathways in the genome of the parasitic copepod.

13. A method for protecting a fish against a parasitic copepod, comprising administering the fish vaccine of any one of claims 1 to 10 to the fish.

14. The fish vaccine of any one of claims 1 to 10, for use for protecting a fish against a parasitic copepod.

15. A bacterial product from a bacteria of the microbiota of a parasitic copepod for use for protecting a fish against the parasitic copepod.
